# EUROPEAN PATENT SPECIFICATION

(11) **EP 4 389 082 B1**
(45) Date of publication and mention of the grant of the patent: **07.10.2026**
(21) Application number: 23218194.1
(22) Date of filing: 19.12.2023
(51) Int. Cl.: A61F 2/38

(54) **CONSTRAINED PROSTHETIC KNEE**
EINGESCHRÄNKTES PROTHESENKNIE
PROTHÈSE DE GENOU CONTRAINTE

(30) Priority: 22.12.2022 US 202263434590 P; 01.12.2023 US 202318526737
(43) Date of publication of application: 26.06.2024
(60) Divisional application: 25177112.7 / 4 582 064
(73) Proprietor: Zimmer, Inc., Warsaw, IN 46580 (US)
(72) Inventor: YOKO, Tim, Granger, 46530 (US); BYRD, Brian D., North Webster, 46555 (US)
(74) Representative: Taor, Simon Edward William

(56) References cited:
- EP-A1- 1 325 718
- EP-A1- 3 127 510
- CN-A- 114 869 547
- US-A1- 2010 174 378

## Description

### CLAIM OF PRIORITY

This application claims the benefit of U.S. Provisional Patent Application Serial No. 63/434,590, filed on December 22, 2022, and U.S. Patent Application Serial No. 18/526,737, filed on December 1, 2023, the benefit of priority of which is claimed hereby.

### TECHNICAL FIELD

The present disclosure generally relates to orthopedic prostheses. More specifically, the present disclosure relates to orthopedic prostheses used in constrained knee arthroplasties.

### BACKGROUND

Orthopedic procedures and prostheses are commonly utilized to repair or replace damaged bone and tissue in the human body. Generally, the knee is formed by the pair of condyles at the distal portion of the femur, the lower surfaces of which bear upon the correspondingly shaped proximal surface plateau of the tibia. The femur and tibia are connected by means of ligaments such as, the posterior cruciate ligament, the lateral collateral ligament, the medial collateral ligament, and the anterior cruciate ligament. These ligaments provide stability to the knee j oint.

Prosthetic knee joints can be considered either constrained or unconstrained. Constrained prosthetic knee systems can include femoral and tibial prostheses, which are mechanically linked or constrained to each other to limit relative movement between the femoral and tibial prostheses. Common mechanisms for such mechanical linkage can include a hinge, band, or other linkage structure. An unconstrained prosthetic knee system includes femoral and tibial prostheses, which are not mechanically linked. An unconstrained knee utilizes the existing ligaments and other soft tissue of the patient to provide joint stability. Constrained prosthetic knees have particular applicability to cases where a patient has experienced ligament loss or the existing ligaments do not provide adequate support and stability to the knee.

CN114869547A discloses a pre-assembled rotary hinge subassembly having an articulating rotary femoral cassette configured to be mechanically engaged to a femoral component by a femoral fastening mechanism, the femoral fastening mechanism is non-axially aligned with the tibial axis of rotation when the knee joint is flexion or extended.

US2010/174378A1 discloses a prosthesis system for replacing a knee joint between a femur and a tibia that can include a femoral component, a tibial component, a bearing, a first yoke, and a first key. The femoral component can include a first condylar portion, a second condylar portion, a first sidewall extending superiorly from the first condylar portion, a second sidewall extending superiorly from the second condylar portion where the first and second sidewalls collectively comprise a first hinge portion. The tibial component can have a bone engaging inferior surface and a bearing engaging superior surface. The bearing can have an inferior surface that engages the bearing engaging surface and a superior femoral engaging surface. The bearing can define an opening. The first yoke can have an inferior portion, a superior portion and a yoke keyway extending therethrough.

EP1325718A1 discloses a tibial component for knee joint prosthesis that has a tibia bearing surface on which slides a bearing with two concave bearing shells to receive a femoral component. There is a rotational guide to define a fixed rotational axis and with cylindrical pins on the tibial bearing face engaged in recesses on the bearing. A radially extending pin can be spaced from the free pin ends.

### OVERVIEW

This disclosure pertains generally to improved constrained knee prostheses, particularly those utilizing a hinge post. Some constrained knee prostheses with hinge posts utilize a design where the femoral component (and hinge post) are free to move generally proximal/distal relative to the tibial baseplate and the tibial bearing component. The inventors of the present disclosure have recognized the need for a system that provides a prosthesis that can be reconfigured for either a mobile bearing system or a fixed bearing system. The system can include components that can be assembled to form a mobile bearing constrained-knee prosthesis and a fixed constrained-knee prosthesis.

The invention is set out in the appended claims.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIGS. 1 and 2 illustrate knee joint structures providing suitable environments in which the constrained prosthesis assemblies in accordance with an example of the present application can be utilized.
FIG. 3 is a perspective view of a constrained knee prosthesis assembly, according to an example of the present application.
FIG. 4 is an exploded view of the constrained knee prosthesis assembly of FIG. 3, according to an example of the present application.
FIG. 5 is a superior view of an example of a constrained knee prosthesis, according to an example of the present application.
FIG. 6 is a partial cross-sectional view of a constrained knee prosthesis assembly taken along indicator 7-7 of FIG. 5, according to an example of the present application.
FIG. 7 is a superior view of an example of a constrained knee prosthesis including a mobile bearing rotated to a first set limit, according to an example of the present application.
FIG. 8 is a superior view of an example of a constrained knee prosthesis including a mobile bearing rotated to a second set limit, according to an example of the present application.
FIG. 9 is a superior view of an example of a constrained knee prosthesis including a fixed bearing, according to an example of the present application.
FIG. 10 is a superior view of an example of a constrained knee prosthesis assembly including a mobile bearing with a press-insert, according to an example of the present application.

### DETAILED DESCRIPTION

The present application relates to a system that includes a constrained knee prosthesis. The system includes a set of components, e.g., a tibial tray or baseplate, a shackle, a femoral component, a hinge post, a poly box, an axle bushing, or the like. The set of components is configured to fit with a tibial bearing component. The system includes a mobile tibial bearing component or a fixed tibial bearing component, which can be substituted at surgeon discretion. The mobile tibial bearing component is configured to limit rotation (or movement) about the at least one axis as compared with the mobile tibial bearing component by engaging with one or more engagement features of the tibial tray. The fixed bearing component is configured to prevent or severely limit rotation (or movement) about at least one axis by contacting with one or more engagement features of the tibial tray.

In another example, the system can include the set of components, a mobile tibial bearing component, and a press-fit insert. In such examples, the press-fit insert can be configured to fill the space between the mobile tibial bearing component and one or more engagement features of the tibial tray to further limit or prevent rotation or motion of the tibial bearing component relative to the tibial bearing tray about the one or more axes.

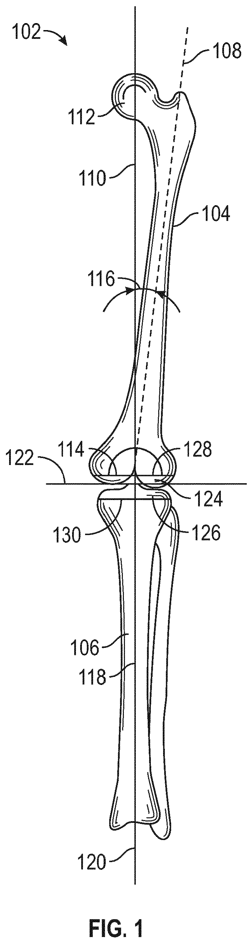

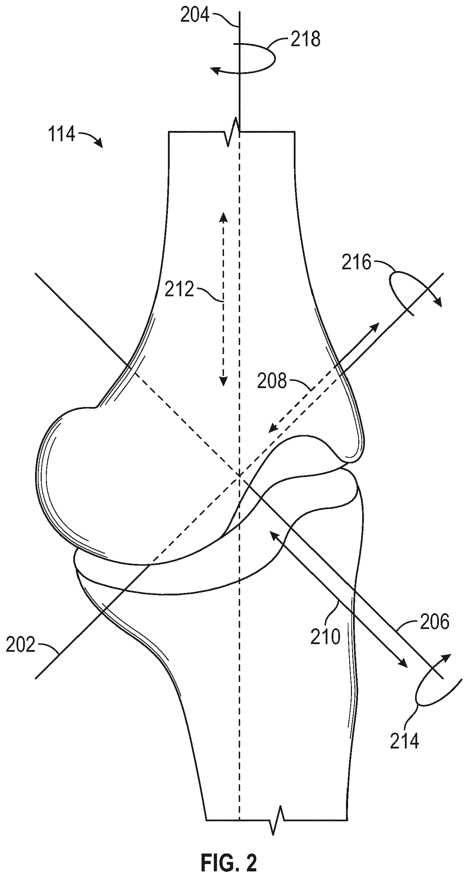

To better understand knee joint replacement procedures, it can be helpful to understand the relationship between bones and bone cuts that can be made to orient various provisional and permanent prosthesis components within a knee joint. FIGS. 1 and 2 illustrate several features of knee joint structures and orientations. In FIG. 1, a frontal view of a lower limb 102, including a femur 104 and a tibia 106, illustrates various lower limb axes. The femur 104 has an anatomic axis 108 that coincides generally with its intramedullary canal. The femur 104 also has a mechanical axis 110, or load axis, running from the center of a femoral head 112 to the center of a knee joint 114. The angle 116 extending between these two axes varies among the patient population but is generally between 5-7 degrees, inclusive. Like the femur 104, the tibia 106 also has an anatomic axis coinciding generally with its intramedullary canal. The mechanical axis 118 of the tibia 106 runs from the center of the knee joint 114 to the center of an ankle region 120 and is generally collinear with its anatomic axis.

A joint line 122, about which the knee joint 114 flexes, is approximately parallel to a line through medial and lateral femoral condyles 124 and to a tibial plateau 126. Although illustrated as perpendicular in FIG. 1, the joint line 122 can extend at a varus or valgus angle relative to the mechanical axes 110 and 118 of the femur 104 and tibia 106, respectively. Normally, during a partial or total knee replacement procedure, portions of a distal end of the femur 104 or a proximal end of the tibia 106 are resected to be parallel or approximately parallel to the joint line 122, and thus perpendicular to the mechanical axes 110 and 118, as indicated at 128 and 130, respectively.

Typical knees can move between an extended state, where a longitudinal axis of the femur 104 and a longitudinal axis of the tibia 106 are essentially parallel, and a flexed state, where the longitudinal axis of the femur 104 and the longitudinal axis of the tibia 106 are angled relative to each other around 140 degrees. In examples, the extended state can be restricted to +/- 10 degrees, and the flexed state can be much less than 140 degrees. For example, the flexed state can include limits where the longitudinal axis of the femur 104 and the longitudinal axis of the tibia 106 are angled relative to each other around 90 to 140 degrees.

FIG. 2 illustrates a closer view of the knee joint 114 and its coordinate system, in which a medial/lateral axis 202 corresponds approximately to the joint line 122 (FIG. 1), a proximal/distal axis 204 corresponds approximately to the mechanical axes 110 and 118 (FIG. 1), and an anterior/posterior axis 206 is approximately normal to the other two axes. The arrows can depict positions along each of these axes, representing the medial/lateral 208, anterior/posterior 210, and proximal/distal 212 positioning of inserted prosthesis components. Rotation about each of these axes can also be depicted by arrows. Rotation about the proximal/distal axis 204 can correspond anatomically to external rotation of a femoral component, while rotation about the anterior/posterior axis 206 and medial/lateral axis 202 can correspond to extension plane slope and varus/valgus angle of a component, respectively. Depending on a position of the proximal tibial cut 130 (FIG. 1) made, a varus/valgus angle 214, extension plane angle 216, external rotation 218, or joint extension gap can be affected. Similarly, a position of the distal femoral cut 128 (FIG. 1) can affect the location of the joint line 122, the extension gap, the varus/valgus angle 214, or the extension plane angle 216.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the terms "proximal" and "distal" should be interpreted as though the patient were standing with the knee joint in extension. The intent is to differentiate the terms "proximal" and "distal" from the terms "anterior" and "posterior." As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior." Similarly, the term "lateral" refers to the opposite direction of "medial." The term "medial/lateral" means medial to lateral or lateral to medial. The term "proximal/distal" means proximal to distal or distal to proximal. The term "anterior/posterior" means anterior to posterior or posterior to anterior.

As used herein, the "periphery" of a tibial baseplate refers to any periphery as viewed in a top plan view, e.g., in a generally transverse anatomical plane. Alternatively, the periphery of a tibial baseplate may be any periphery as viewed in the bottom plan view, e.g., in a generally transverse plane and looking at the distal surface adapted to contact a resected proximal surface of a tibial bone.

The term "micromotion" refers to the small motions that may exist between prosthesis components, such as between the tibial baseplate and capture element, respectively, upon application of force. Such small motions may occur as a result of material deformation in one or both of the interacting components, or may result from slight spaces or clearances therebetween, for example. Micromotion is distinguished from larger movements of components such as the proximal/distal movement of the femoral component relative to the tibial baseplate and the tibial bearing component.

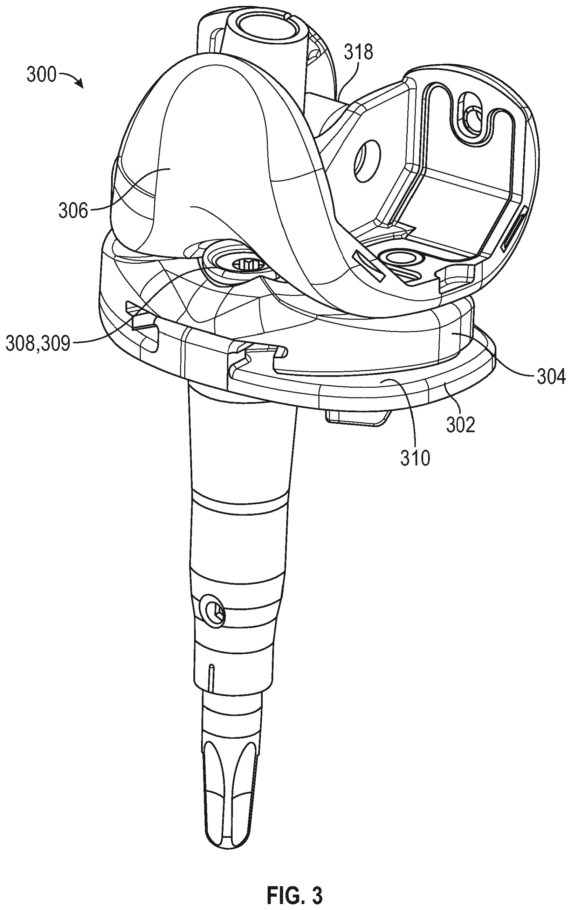

FIG. 3 shows a prosthesis assembly 300 for a constrained knee. The prosthesis assembly 300 includes include a tibial baseplate 302, a tibial bearing component 304 (sometime called a meniscal component, poly, articular component or bearing), a femoral component 306 and a hinge post 308.

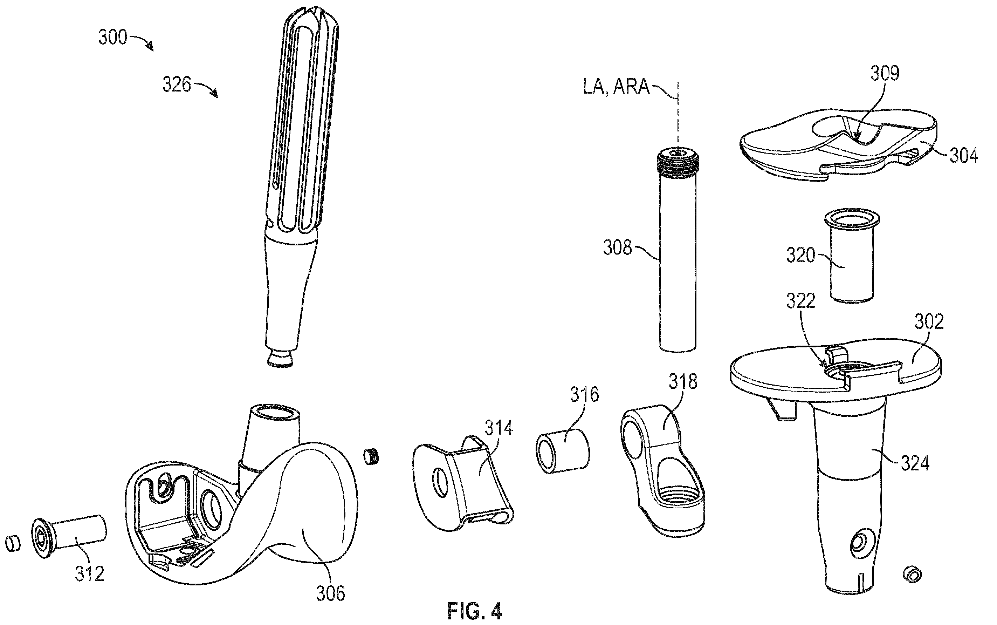

The tibial bearing component 304 is configured to be coupled to and can be positioned atop a proximal surface 310 of the tibial baseplate 302. The tibial bearing component 304 can be formed of polymer material such as Ultra-High-Molecular-Weight-Polyethylene ("UHMWPE"), or the like. The tibial bearing component 304 can be configured to articulate with the femoral component 306 through knee joint flexion and extension as known in the art. The prosthesis assembly 300 has the femoral component 306 and the tibial baseplate 302 mechanically linked to one another. This is accomplished by the hinge post 308 and other components further illustrated and discussed in FIG. 4. The hinge post 308 is coupled to the femoral component 306 and is received within a recess 309 of the tibial bearing component 304 and a recess 322 (FIG. 4) of the tibial baseplate 302.

FIG. 4 shows an exploded view of the prosthesis assembly 300, the tibial baseplate 302, the tibial bearing component 304, the femoral component 306, the hinge post 308 and further illustrates a hinge axle 312, poly box 314, axle bushing 316, shackle 318 and a bushing.

The hinge post 308 is connected to femoral component 306 via the shackle 318, the axle bushing 316, and the hinge axle 312. A distal portion of the shackle 318 is received in the recess 309 in the tibial bearing component 304 and the distal portion is threaded or otherwise connected to the hinge post 308. The hinge post 308 extends distally through the recess 309 of the tibial bearing component 304 and is received in a recess 322 of the tibial baseplate 302. The recess 322 of the tibial baseplate 302 that receives the hinge post 308 can at least partially be formed by a keel 324 of the tibial baseplate 302. The hinge post 308 can be moveable, e.g., rotatable or capable of distraction, relative to the tibial bearing component 304 or the tibial baseplate 302. The hinge post 308 can be rotatably connected to femoral component 306 via the hinge axle 312. Thus, a longitudinal axis LA that defines a centerline of the hinge post 308 can define an axis of rotation/articulation ARA for the knee joint as the femoral component 306 and the tibial baseplate 302 are mechanically linked.

When assembled, the shackle 318 can be placed between opposing walls of poly box 314. When assembled on the hinge axle 312, the axle bushing 316 additionally resides within an aperture on a proximal portion of the shackle 318. The shackle 318 and hinge post 308 can be formed from suitable materials such as a titanium alloy, a cobalt-chromium alloy, or the like, while the axle bushing 316 and the poly box 314 can be formed from different materials such as plastic, e.g., UHMWPE. The axle bushing 316 can act as a bearing between the shackle 318 and the hinge axle 312. The poly box 314 can act as a bearing between the femoral component 306 and the shackle 318.

The prosthesis assembly 300 of FIG. 4 shows a system 326 of components where distraction of the knee is not limited by a capture element or other feature of the prosthesis assembly 300. Thus, the system 326 provides components that, when assembled, are configured for full distraction. Thus, soft tissue of the knee is relied upon to limit distraction between the femoral component 306 and the tibial baseplate 302 and the tibial bearing component 304 as discussed previously. The bushing 320 can be configured to insert into the recess 322 of at least the tibial baseplate 302. The bushing 320 can also insert into or through the recess 309 (FIG. 3) of the tibial bearing component 304 in some examples. The bushing 320 can be configured to receive at least a portion of the hinge post 308. The bushing 320 can act as a bearing between the hinge post 308 and the tibial baseplate 302. The hinge post 308 can be moveable generally proximal/distal relative to the bushing 320.

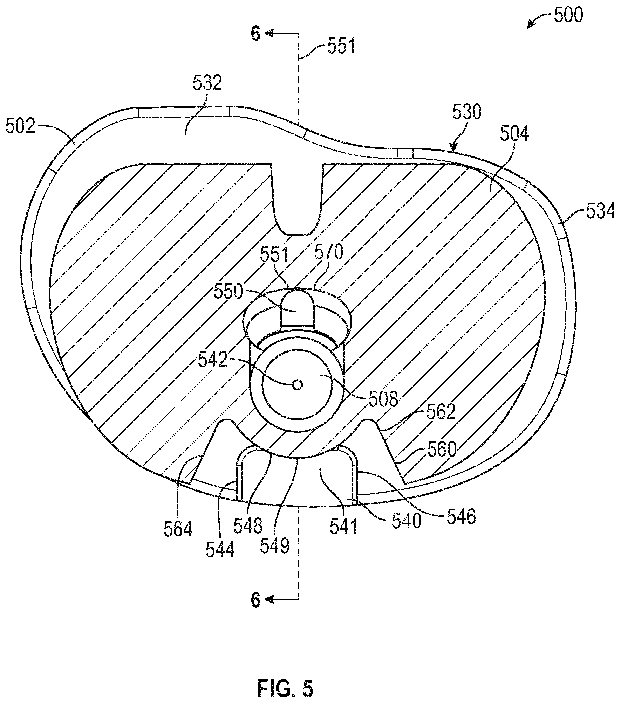

FIG. 5 is a superior view of an example of a mobile bearing prosthesis, e.g., a prosthesis assembly 500, according to an example of the present application. The prosthesis assembly 500 is configured for implant in a constrained knee patient. In examples, the prosthesis assembly 500 includes a tibial baseplate 502, a tibial insert or tibial bearing component (tibial bearing component 504), and a hinge post 508.

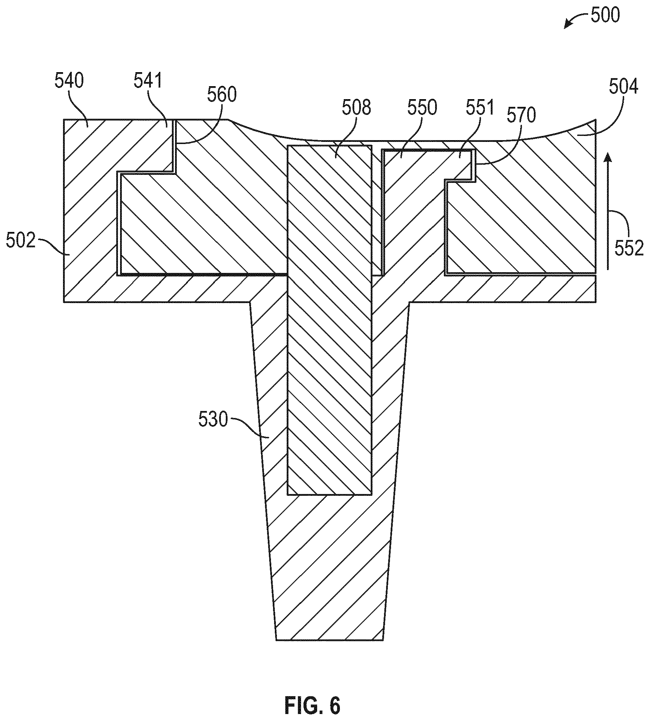

The tibial baseplate 502, similar to the tibial baseplate 302 from FIG. 3, can be configured to be inserted into a tibia, e.g., tibia 106 from FIG. 1, of a patient. The tibial baseplate 502 includes a distal surface 530 (shown in FIG. 6) and a proximal surface 532. The proximal surface 532 is opposite the distal surface 530, and a periphery 534 of the tibial baseplate 502 extends between the distal surface 530 and the proximal surface 532. The proximal surface 532 can include a recess that is configured to receive the hinge post 508. The proximal surface 532 can also include a first engagement feature 540 and a second engagement feature 550.

The tibial bearing component 504, similar to the tibial bearing component 304 from FIG. 3, is configured to be coupled to the tibial baseplate 502 and to rotate about at least a first axis 542, e.g., external rotation 218 around distal axis 204 of FIG. 2. The first axis 542 extends through a centerline of the hinge post 508. The tibial bearing component 504 can include a third engagement feature 560, and a fourth engagement feature 570.

The first engagement feature 540 is configured to limit rotation or movement of the tibial bearing component 504 with relation to the tibial baseplate 502 about at least the first axis 542. The first engagement feature 540 can include a protrusion 541. The protrusion 541 can extend from the proximal surface 532 of the tibial baseplate 502. Alternatively, other examples of the present application contemplate that the first engagement feature 540 could be a slot, aperture, or other recess. In the example shown in FIG. 5, the protrusion 541 can extend from an anterior portion of the proximal surface 532 adjacent the periphery 534 and can extend away from the distal surface 530. In another example, the protrusion 541 can extend from a posterior portion, medial portion, lateral portion, or any other portion of the proximal surface 532 that can limit rotation or movement of the tibial bearing component 504 with relation to the tibial baseplate 502 about at least the first axis 542, or the like. The first engagement feature 540 can include a first edge 544, a second edge 546, and a third edge 548.

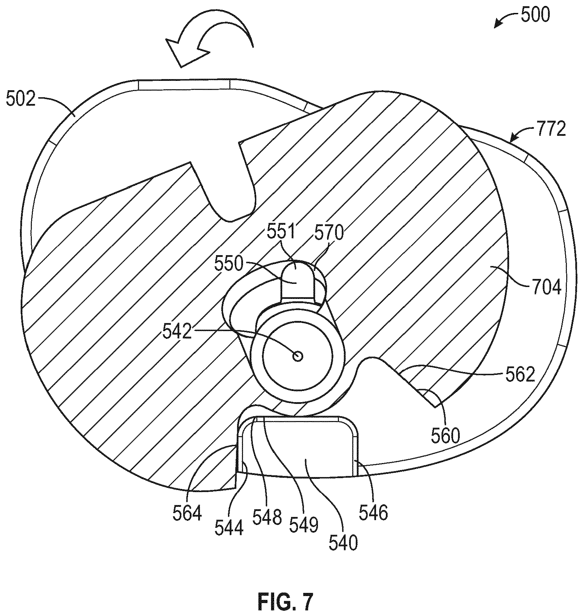

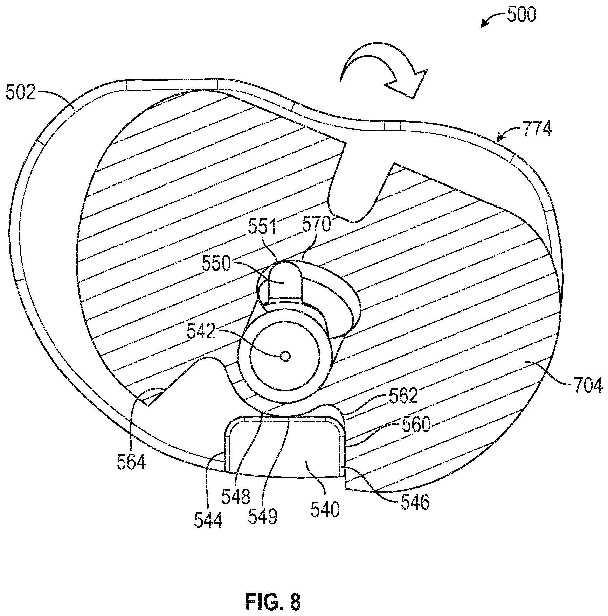

The first edge 544 can extend posteriorly from the periphery of the tibial baseplate 502. The second edge 546 can also extend posteriorly from the periphery of the tibial baseplate 502. The third edge 548 can extend from the first edge 544 to the second edge 546. The third edge 548 can also include a concave profile 549 such that a thickness of the first engagement feature 540 decreases in a middle portion as compared with the first edge 544 or the second edge 546. In examples, the first edge 544 or the second edge 546 can engage with the tibial bearing component 504 to limit further rotation of the tibial bearing component 504 relative to the tibial baseplate 502 about the first axis 542. The interactions between the tibial bearing component 504 and the first edge 544, the second edge 546, and the third edge 548 of the first engagement feature 540 will be discussed in more detail below with reference to FIGS. 7 and 8.

The third engagement feature 560 can be configured to engage with the first engagement feature 540 of the tibial bearing component 504 to limit rotation of the tibial bearing component 504 relative to the tibial baseplate 502 about at least the first axis 542. The third engagement feature 560 can be an anterior recess that can include a first internal wall 562 and a second internal wall 564. For example, the third engagement feature 560 can be configured to contact the third edge 548 of the first engagement feature 540 upon coupling the tibial bearing component 504 onto the tibial baseplate 502. The contact between the third engagement feature 560 and the third edge 548 can help keep the tibial bearing component 504 on the tibial baseplate 502.

The second internal wall 564 of the third engagement feature 560 can engage with the first edge 544. The first internal wall 562 of the third engagement feature 560 can engage with the second edge 546. Thus, the first internal wall 562 and the second internal wall 564 of the third engagement feature 560 can engage with the second edge 546 and the first edge 544, respectively, to limit rotation in either clockwise or counterclockwise directions of the tibial bearing component 504 relative to the tibial baseplate 502 about the first axis 542. As shown in FIG. 5, the third engagement feature 560 can be formed on an anterior portion of the tibial bearing component 504 adjacent the periphery 534 thereof. In another example, the third engagement feature 560 can be formed in any other portion of the tibial bearing component 504 such that the third engagement feature 560 can limit rotation of the tibial bearing component 504 relative to the tibial baseplate 502 about the first axis 542. The interactions between the third engagement feature 560 and the first engagement feature 540 will be discussed in more detail below with reference to FIGS. 7 and 8.

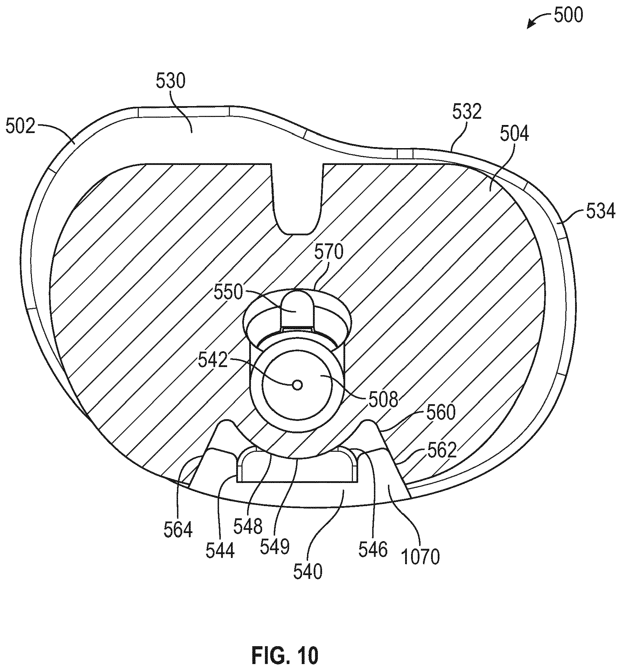

The second engagement feature 550 can be configured to limit movement of the tibial bearing component 504 with relation to the tibial baseplate 502 along at least second direction 552. The movement of the tibial bearing component 504 along the second direction 552, can be limited by engagement of the second engagement feature 550 and the tibial bearing component 504. As shown in FIG. 5, the second engagement feature 550 can include a protrusion 551 that extends from a central portion of the proximal surface 532 such as adjacent but spaced immediately posterior of the recess that receives the hinge post 508. In another example, the second engagement feature 550 can extend from a posterior, medial, lateral, or any other portion of the proximal surface 532 such that the second engagement feature 550 can limit movement of the tibial bearing component 504 relative to the tibial baseplate 502 along the second direction 552, or the like. The interactions between the tibial bearing component 504 and the second engagement feature 550 will be discussed in more detail below with reference to FIGS. 10 and 8.

The fourth engagement feature 570 can be configured to engage with the second engagement feature 550 of the tibial bearing component 504 to limit movement of the tibial bearing component 504 relative to the tibial baseplate 502 along at least the second direction 552. The fourth engagement feature 570 can be a wall of a recess formed in the tibial bearing component 504. The recess can be centralized and can communicate to receive the hinge post 508 in addition to the second engagement feature 550. The interactions between the fourth engagement feature 570 and the second engagement feature 550 will be discussed in more detail below with reference to FIGS. 10 and 8.

As shown in FIG. 5, the first engagement feature 540 and the second engagement feature 550 of the tibial baseplate 502 can be protrusions, and the third engagement feature 560 and the fourth engagement feature 570 of the tibial bearing component 504 can be walls of recesses. **In** another example, the first engagement feature 540 and the second engagement feature 550 of the tibial baseplate 502 can be recesses, ridges, grooves, or any other formation formed in the surface of the tibial baseplate 502, or the like, and the third engagement feature 560 and the fourth engagement feature 570 can be protrusions, ridges, extensions, or any other formation that extends from the surface of the tibial bearing component 504, or the like.

FIG. 6 is a partial cross-sectional view of a constrained knee prosthesis assembly, e.g., the prosthesis assembly 500, taken along indicators 7-7 of FIG. 5, according to an example of the present application. As discussed above, and shown in FIG. 6, the tibial bearing component 504 is configured to be coupled to the tibial baseplate 502. When the tibial bearing component 504 is coupled to the tibial baseplate 502, the third engagement feature 560 and the fourth engagement feature 570 of the tibial bearing component 504 can engage with the first engagement feature 540 and the second engagement feature 550, respectively.

As shown in FIG. 6, the third engagement feature 560 can include a ridge that can be slid under a surface of the protrusion 541 that faces the distal surface 530 of the tibial baseplate 502. The fourth engagement feature 570 can also include a ridge that can be slid under a surface of the protrusion 551 that faces the distal surface 530 of the tibial baseplate 502. As such, during the coupling of the tibial bearing component 504 to the tibial baseplate 502 the ridge of the third engagement feature 560 can be slid under the protrusion 541, and then the ridge of the fourth engagement feature 570 can be slid under the protrusion 551. During the coupling, the first engagement feature 540 and the second engagement feature 550 can be configured such that they flex away from the hinge post 508 and toward the hinge post 508, respectively, to make the coupling of the tibial bearing component 504 to the tibial baseplate 502 easier. Once coupled, the protrusion 541 and the protrusion 551 can engage with the ridges of the third engagement feature 560 and the fourth engagement feature 570, respectively, to prevent the tibial bearing component 504 from moving in the second direction 552 relative to the tibial baseplate 502.

FIG. 7 is a superior view of an example of a constrained knee prosthesis, e.g., the prosthesis assembly 500, including a mobile bearing 704 rotated to a first set limit, according to an example of the present application. The mobile bearing 704, similar to the tibial bearing component 504 (FIG. 5), is configured to couple with a tibial baseplate 502. As discussed above, the mobile bearing 704 is configured to rotate about at least a first axis 542 that is along the centerline of the hinge post 508. As shown in FIG. 7, the mobile bearing 704 can rotate into orientation 772.

In orientation 772, the mobile bearing 704 can rotate counterclockwise in relation to the tibial baseplate 502 about the first axis 542. For example, the mobile bearing 704 can rotate counterclockwise with relation to the tibial baseplate 502 about the first axis 542 until the second internal wall 564 of third engagement feature 560 contacts the first edge 544 of the first engagement feature 540. In such an example, the contact between the second internal wall 564 and the first edge 544 can prevent the mobile bearing 704 from rotating further about the first axis 542 in relation to the tibial baseplate 502. As shown in FIG. 7, the second engagement feature 550 can engage with the fourth engagement feature 570 to prevent the mobile bearing 704 from rotating further about the first axis 542 in relation to the tibial baseplate 502.

FIG. 8 is a superior view of an example of a constrained knee prosthesis, e.g., the prosthesis assembly 500, including mobile bearing 704 rotated clockwise to a second set limit, according to an example of the present application. The mobile bearing 704 can rotate clockwise about the first axis 542 with respect to the tibial baseplate 502 to any degree between the orientation 772 and an orientation 774. The prosthesis assembly 500 with the mobile bearing 704 is typically biased more toward a neutral position between the orientation 772 and the orientation 774.

In orientation 774 of FIG. 7, the mobile bearing 704 can rotate with relation to the tibial baseplate 502 about the first axis 542. For example, the mobile bearing 704 can rotate with relation to the tibial baseplate 502 about the first axis 542 until the first internal wall 562 of the third engagement feature 560 contacts the second edge 546 of the first engagement feature 540. In such an example, the contact between the first internal wall 562 and the second edge 546 can prevent the mobile bearing 704 from rotating further about the first axis 542 in relation to the tibial baseplate 502.

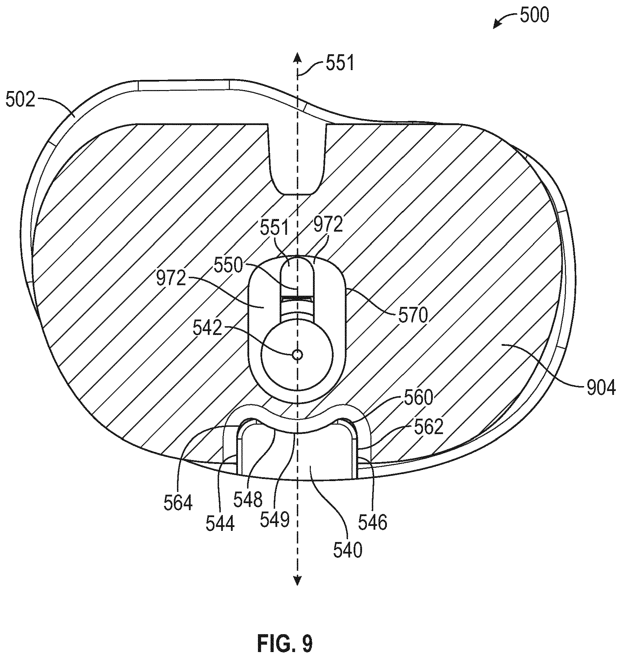

FIG. 9 is a superior view of an example of a constrained knee prosthesis, e.g., the prosthesis assembly 500, including a fixed bearing 904, according to an example of the present application. The fixed bearing 904, e.g., the tibial bearing component 504 (FIG. 5), is configured to couple with a tibial baseplate 502. As discussed above, the fixed bearing 904 is configured to prevent rotation or movement of the fixed bearing 904 with relation to the tibial baseplate 502 about the first axis 542 and the second direction 552.

The third engagement feature 560 of the fixed bearing 904 is configured to surround and contact the first engagement feature 540 of the tibial baseplate 502 such as the prevent movement of the fixed bearing 904 relative to the tibial baseplate 502 about the first axis 542. For example, the recess of the third engagement feature 560 that includes first internal wall 562 and second internal wall 564 along the anterior portion of the fixed bearing 904 is smaller than the recess of the third engagement feature 560 on the mobile bearing 704. As such, the first internal wall 562 and the second internal wall 564 of the third engagement feature 560 of the fixed bearing 904 are configured to contact the second edge 546 and the first edge 544, respectively as the fixed bearing 904 is coupled to the tibial baseplate 502. Therefore, the first internal wall 562 and the second internal wall 564 of the fixed bearing 904 prevent rotation of the fixed bearing 904 relative to the tibial baseplate 502 about the first axis 542.

The fourth engagement feature 570 of the fixed bearing 904 is configured to surround and contact the second engagement feature 550 of the tibial baseplate 502 to prevent movement of the fixed bearing 904 relative to the tibial baseplate 502 about the second axis 551. The fourth engagement feature 570 can include flanges, projections, tangs, or ribs (ribs 972). The ribs 972 are configured to surround opposing sides of the second engagement feature 550 and the protrusion 551 to help prevent rotation of the fixed bearing 904 relative to the tibial baseplate 502 about the first axis 542.

FIG. 10 is a superior view of an example of a constrained knee prosthesis assembly, e.g., the prosthesis assembly 500, including a mobile bearing, e.g., the mobile bearing 704, with a press fitting 1070, according to an example of the present application.

As discussed above, the prosthesis assembly 500 can be used as a mobile bearing prosthesis by coupling the mobile bearing 704 to the tibial baseplate 502. The prosthesis assembly 500 can be used as a fixed bearing prosthesis by coupling the fixed bearing 904 to the tibial baseplate 502. In another example, the prosthesis assembly 500 can be made a fixed prosthesis by using a press fitting 1070. In examples, the press fitting 1070 can be configured to be inserted into the third engagement feature 560 of the mobile bearing 704. In this example, the press fittings can be configured to fill the space between the first internal wall 562 of the third engagement feature 560 and the second edge 546 of the first engagement feature 540 and configured to fill the gap between the second internal wall 564 of the third engagement feature 560 and the first edge 544 of the first engagement feature 540. Therefore, the press fitting 1070 can effectively turn the mobile bearing 704 into a fixed bearing. As such, the mobile bearing 704 can be coupled onto the tibial baseplate 502 then the press fitting 1070 can be installed onto the mobile bearing 704 such that the press fittings engage with the third engagement feature 560.

As shown in FIG. 10, the first engagement feature 540 can include a slot that extends from a periphery of the mobile bearing 704. The slot can receive the press fitting 1070 such that the press fitting 1070 slides within the slot and surrounds the first engagement feature 540. In the examples of FIG. 10, the press fitting 1070 is configured to fill the entire space between the first edge 544 of the first engagement feature 540 and the second internal wall 564 of the third engagement feature 560 and to fill the entire space between the second edge 546 of the first engagement feature 540 and the first internal wall 562 of the third engagement feature 560. In another example, the press fitting 1070 can be configured to fill any portion of the space between the first edge 544 of the first engagement feature 540 and the second internal wall 564 of the third engagement feature 560 and to fill the space between the second edge 546 of the first engagement feature 540 and the first internal wall 562 of the third engagement feature 560.

The above-detailed description includes references to the accompanying drawings, which form a part of the detailed description. The drawings show, by way of illustration, specific embodiments that may be practiced. These embodiments are also referred to herein as "examples." Such examples may include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

. In the event of inconsistent usages between this document and those documents so referenced, the usage in the reference(s) should be considered supplementary to that of this document; for irreconcilable inconsistencies, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In the appended claims, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 10% of the numerical value of the number with which it is being used. Therefore, about 50% means in the range of 45%-55%. Numerical ranges recited herein by endpoints include all numbers and fractions subsumed within that range, e.g., 1 to 5 includes 1, 1.5, 2, 2.75, 3, 3.90, 4, 4.24, and 5. Similarly, numerical ranges recited herein by endpoints include subranges subsumed within that range, e.g., 1 to 5 includes 1-1.5, 1.5-2, 2-2.75, 2.75-3, 3-3.90, 3.90-4, 4-4.24, 4.24-5, 2-5, 3-5, 1-4, and 2-4. It is also to be understood that all numbers and fractions thereof are presumed to be modified by the term "about."

The above description is intended to be illustrative, and not restrictive. For example, the above-described examples (or one or more aspects thereof) may be used in combination with each other. Other embodiments may be used, such as by one of ordinary skill in the art upon reviewing the above description. The Abstract is to allow the reader to quickly ascertain the nature of the technical disclosure and is submitted with the understanding that it will not be used to interpret or limit the scope or meaning of the claims. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment. Thus, the following claims are hereby incorporated into the Detailed Description, with each claim standing on its own as a separate embodiment. The scope of the embodiments should be determined with reference to the appended claims, along with the full scope of equivalents to which such claims are entitled.

## Claims

1. A system including a prosthesis assembly (500) for a constrained knee comprising:
a set of components including:
a femoral component;
a shackle component extending between a first end portion and a second end portion;
a hinge axle configured to secure the femoral component to the first end portion of the shackle component;
a tibial baseplate (502) having a distal surface (530), a proximal surface (532) opposite the distal surface (530), a periphery (534) extending between the proximal surface (532), the tibial baseplate (502) including a first engagement feature (540); and
a hinge post (508) extending between a first end section and a second end section, the first end section configured to be attached to the second end portion of the shackle component, the second end section configured to be at least partially received by a recess in the tibial baseplate (502);
a mobile bearing (704) configured to couple with the tibial baseplate (502) and to rotate about at least a first axis (542) approximated by a centerline of the hinge post (508), the rotation about at least the first axis (542) is limited by at least the first engagement feature (540) of the tibial baseplate (502); and
a fixed bearing (904) configured to be coupled to the tibial baseplate (502), the fixed bearing (904) is restrained from rotating about the first axis (542) by at least the first engagement feature (540) of the tibial baseplate (502);
wherein the set of components is configured to be assembled with either the mobile bearing (704) or the fixed bearing (904).

2. The system of claim 1, wherein the first engagement feature (540) of the tibial baseplate (502) includes a first protrusion (541) extending from an anterior portion of the proximal surface (532) away from the distal surface (530), and wherein the first protrusion (541) includes:
a first edge (544) extending posteriorly from the periphery (534) of the tibial baseplate (502);
a second edge (546) extending posteriorly from the periphery (534) of the tibial baseplate (502); and
a third edge (548) extending from the first edge (544) to the second edge (546), the third edge (548) includes a concave profile (549).

3. The system of claim 2, wherein the tibial baseplate (502) further comprises a second engagement feature (550).

4. The system of claim 3, wherein the second engagement feature (550) includes a second protrusion (551) extending from a central portion of the proximal surface (532) and away from the distal surface (530).

5. The system of claim 4, wherein the mobile bearing (704) comprises a third engagement feature (560) configured to contact the third edge (548) of the first engagement feature (540) upon coupling of the mobile bearing (704) with the tibial baseplate (502), the third engagement feature (560) is also configured to engage with the first edge (544) or the second edge (546) of the first engagement feature (540) upon rotation of the mobile bearing (704) about at least the first axis (542).

6. The system of claim 5, wherein engagement of the third engagement feature (560) with the first edge (544) and the second edge (546) of the first engagement feature (540) limits the rotation of the mobile bearing (704).

7. The system of any of claims 5-6, wherein the third engagement feature (560) is formed in an anterior portion of the mobile bearing (704).

8. The system of any of claims 4-7, wherein the mobile bearing (704) comprises a fourth engagement feature (570) configured to engage with the second engagement feature (550) of the tibial baseplate (502) upon rotation of the mobile bearing (704) about a second axis relative to the tibial baseplate (502), and wherein engagement of the fourth engagement feature (570) with the second engagement feature (550) limits the rotation of the mobile bearing (704) about the second axis.

9. The system of claim 8, wherein the fourth engagement feature (570) is formed in the mobile bearing (704) adjacent to a recess configured to receive the hinge post (508).

10. The system of claim 9, wherein the fourth engagement feature (570) is posterior to the recess configured to receive the hinge post (508).

11. The system of any of claims 8-10, wherein the rotation of the mobile bearing (704) about the second axis includes adduction and abduction of the prosthesis assembly.

12. The system of any of claims 3-11, wherein the fixed bearing (904) is configured to engage with the first engagement feature (540) of the tibial baseplate (502) upon coupling of the fixed bearing (904) with the tibial baseplate (502).

13. The system of claim 12, wherein the fixed bearing (904) comprises a third engagement feature (560) that contacts the first edge (544), the second edge (546), and the third edge (548) of the first engagement feature (540) to prevent rotation of the mobile bearing (704) about at least the first axis (542).

14. The system of any of claims 12-13, wherein the fixed bearing (904) comprises a fourth engagement feature (570) formed in a central portion of the fixed bearing (904), the fourth engagement feature (570) contacts the second engagement feature (550) of the tibial baseplate (502) upon coupling of the fixed bearing (904) with the tibial baseplate (502).

15. The system of claim 14, wherein the fourth engagement feature (570) includes a pair of ribs that engage the second engagement feature (550) on opposing sides thereof.

## Patentansprüche

1. System, das eine Prothesenanordnung (500) für ein eingeschränktes Knie beinhaltet, umfassend:
einen Satz von Komponenten, der Folgendes beinhaltet:
eine Femurkomponente;
eine Schäkelkomponente, die sich zwischen einem ersten Endabschnitt und einem zweiten Endabschnitt erstreckt;
eine Gelenkachse, die dazu konfiguriert ist, die Femurkomponente an dem ersten Endabschnitt der Schäkelkomponente zu sichern;
eine Tibia-Basisplatte (502) mit einer distalen Oberfläche (530), einer proximalen Oberfläche (532) gegenüber der distalen Oberfläche (530), einer Peripherie (534), die sich zwischen der proximalen Oberfläche (532) erstreckt, wobei die Tibia-Basisplatte (502) ein erstes Eingriffsmerkmal (540) beinhaltet; und
einen Scharnierzapfen (508), der sich zwischen einem ersten Endabschnitt und einem zweiten Endabschnitt erstreckt, wobei der erste Endabschnitt dazu konfiguriert ist, an dem zweiten Endabschnitt der Schäkelkomponente befestigt zu werden, wobei der zweite Endabschnitt dazu konfiguriert ist, mindestens teilweise von einer Aussparung in der Tibia-Basisplatte (502) aufgenommen zu werden;
ein bewegliches Lager (704), das dazu konfiguriert ist, mit der Tibia-Basisplatte (502) zu koppeln und sich um mindestens eine erste Achse (542) zu drehen, die durch eine Mittellinie des Scharnierzapfens (508) angenähert ist, wobei die Drehung um mindestens die erste Achse (542) durch mindestens das erste Eingriffsmerkmal (540) der Tibia-Basisplatte (502) begrenzt ist; und
ein Festlager (904), das dazu konfiguriert ist, an die Tibia-Basisplatte (502) gekoppelt zu sein, wobei das Festlager (904) durch mindestens das erste Eingriffsmerkmal (540) der Tibia-Basisplatte (502) daran eingeschränkt wird, sich um die erste Achse (542) zu drehen;
wobei der Satz von Komponenten dazu konfiguriert ist, entweder mit dem beweglichen Lager (704) oder dem Festlager (904) zusammengebaut zu werden.

2. System nach Anspruch 1, wobei das erste Eingriffsmerkmal (540) der Tibia-Basisplatte (502) einen ersten Vorsprung (541) beinhaltet, der sich von einem vorderen Abschnitt der proximalen Oberfläche (532) weg von der distalen Oberfläche (530) erstreckt, und wobei der erste Vorsprung (541) Folgendes beinhaltet:
eine erste Kante (544), die sich von der Peripherie (534) der Tibia-Basisplatte (502) nach hinten erstreckt;
eine zweite Kante (546), die sich von der Peripherie (534) der Tibia-Basisplatte (502) nach hinten erstreckt; und
eine dritte Kante (548), die sich von der ersten Kante (544) zu der zweiten Kante (546) erstreckt, wobei die dritte Kante (548) ein konkaves Profil (549) beinhaltet.

3. System nach Anspruch 2, wobei die Tibia-Basisplatte (502) ferner ein zweites Eingriffsmerkmal (550) umfasst.

4. System nach Anspruch 3, wobei das zweite Eingriffsmerkmal (550) einen zweiten Vorsprung (551) beinhaltet, der sich von einem zentralen Abschnitt der proximalen Oberfläche (532) und weg von der distalen Oberfläche (530) erstreckt.

5. System nach Anspruch 4, wobei das bewegliche Lager (704) ein drittes Eingriffsmerkmal (560) umfasst, das dazu konfiguriert ist, die dritte Kante (548) des ersten Eingriffsmerkmals (540) bei Kopplung des beweglichen Lagers (704) mit der Tibia-Basisplatte (502) zu berühren, wobei das dritte Eingriffsmerkmal (560) auch dazu konfiguriert ist, mit der ersten Kante (544) oder der zweiten Kante (546) des ersten Eingriffsmerkmals (540) bei Drehung des beweglichen Lagers (704) um mindestens die erste Achse (542) in Eingriff zu treten.

6. System nach Anspruch 5, wobei der Eingriff des dritten Eingriffsmerkmals (560) mit der ersten Kante (544) und der zweiten Kante (546) des ersten Eingriffsmerkmals (540) die Drehung des beweglichen Lagers (704) begrenzt.

7. System nach einem der Ansprüche 5 bis 6, wobei das dritte Eingriffsmerkmal (560) in einem vorderen Abschnitt des beweglichen Lagers (704) ausgebildet ist.

8. System nach einem der Ansprüche 4 bis 7, wobei das bewegliche Lager (704) ein viertes Eingriffsmerkmal (570) umfasst, das dazu konfiguriert ist, mit dem zweiten Eingriffsmerkmal (550) der Tibia-Basisplatte (502) bei Drehung des beweglichen Lagers (704) um eine zweite Achse relativ zu der Tibia-Basisplatte (502) in Eingriff zu treten, und wobei der Eingriff des vierten Eingriffsmerkmals (570) mit dem zweiten Eingriffsmerkmal (550) die Drehung des beweglichen Lagers (704) um die zweite Achse begrenzt.

9. System nach Anspruch 8, wobei das vierte Eingriffsmerkmal (570) in dem beweglichen Lager (704) benachbart zu einer Aussparung ausgebildet ist, die dazu konfiguriert ist, den Scharnierzapfen (508) aufzunehmen.

10. System nach Anspruch 9, wobei sich das vierte Eingriffsmerkmal (570) hinter der Aussparung befindet, die dazu konfiguriert ist, den Scharnierzapfen (508) aufzunehmen.

11. System nach einem der Ansprüche 8 bis 10, wobei die Drehung des beweglichen Lagers (704) um die zweite Achse Adduktion und Abduktion der Prothesenanordnung beinhaltet.

12. System nach einem der Ansprüche 3 bis 11, wobei das Festlager (904) dazu konfiguriert ist, mit dem ersten Eingriffsmerkmal (540) der Tibia-Basisplatte (502) bei Kopplung des Festlagers (904) mit der Tibia-Basisplatte (502) in Eingriff zu treten.

13. System nach Anspruch 12, wobei das Festlager (904) ein drittes Eingriffsmerkmal (560) umfasst, das die erste Kante (544), die zweite Kante (546) und die dritte Kante (548) des ersten Eingriffsmerkmals (540) berührt, um eine Drehung des beweglichen Lagers (704) um mindestens die erste Achse (542) zu verhindern.

14. System nach einem der Ansprüche 12 bis 13, wobei das Festlager (904) ein viertes Eingriffsmerkmal (570) umfasst, das in einem zentralen Abschnitt des Festlagers (904) ausgebildet ist, wobei das vierte Eingriffsmerkmal (570) das zweite Eingriffsmerkmal (550) der Tibia-Basisplatte (502) bei Kopplung des Festlagers (904) mit der Tibia-Basisplatte (502) berührt.

15. System nach Anspruch 14, wobei das vierte Eingriffsmerkmal (570) ein Paar Rippen beinhaltet, die das zweite Eingriffsmerkmal (550) auf gegenüberliegenden Seiten davon in Eingriff nehmen.

## Revendications

1. Système comprenant un ensemble prothèse (500) pour un genou contraint comprenant :
un ensemble de composants comprenant :
un composant fémoral ;
un composant d'étrier s'étendant entre une première partie d'extrémité et une seconde partie d'extrémité ;
un axe de charnière conçu pour fixer le composant fémoral à la première partie d'extrémité du composant d'étrier ;
une embase tibiale (502) comportant une surface distale (530), une surface proximale (532) opposée à la surface distale (530), une périphérie (534) s'étendant entre la surface proximale (532), l'embase tibiale (502) comprenant un premier élément de mise en prise (540) ; et
un montant de charnière (508) s'étendant entre une première section d'extrémité et une seconde section d'extrémité, la première section d'extrémité étant conçue pour être fixée à la seconde partie d'extrémité du composant d'étrier, la seconde section d'extrémité étant conçue pour être au moins partiellement reçue par un évidement dans l'embase tibiale (502) ;
un insert mobile (704) conçu pour être couplé à l'embase tibiale (502) et pour tourner autour d'au moins un premier axe (542) correspondant approximativement à une ligne médiane du montant de charnière (508), la rotation autour d'au moins le premier axe (542) est limitée par au moins le premier élément de mise en prise (540) de l'embase tibiale (502) ; et
un insert fixe (904) conçu pour être couplé à l'embase tibiale (502), l'insert fixe (904) est empêché de tourner autour du premier axe (542) par au moins le premier élément de mise en prise (540) de l'embase tibiale (502) ;
dans lequel l'ensemble de composants est conçu pour être assemblé soit avec l'insert mobile (704) soit avec l'insert fixe (904).

2. Système de la revendication 1, dans lequel le premier élément de mise en prise (540) de l'embase tibiale (502) comprend une première saillie (541) s'étendant à partir d'une partie antérieure de la surface proximale (532) à l'écart de la surface distale (530), et dans lequel la première saillie (541) comprend :
un premier bord (544) s'étendant postérieurement à partir de la périphérie (534) de l'embase tibiale (502) ;
un deuxième bord (546) s'étendant postérieurement à partir de la périphérie (534) de l'embase tibiale (502) ; et
un troisième bord (548) s'étendant du premier bord (544) au deuxième bord (546), le troisième bord (548) comprend un profil concave (549).

3. Système de la revendication 2, dans lequel l'embase tibiale (502) comprend en outre un deuxième élément de mise en prise (550).

4. Système de la revendication 3, dans lequel le deuxième élément de mise en prise (550) comprend une seconde saillie (551) s'étendant à partir d'une partie centrale de la surface proximale (532) et à l'écart de la surface distale (530).

5. Système de la revendication 4, dans lequel l'insert mobile (704) comprend un troisième élément de mise en prise (560) conçu pour venir en contact avec le troisième bord (548) du premier élément de mise en prise (540) lors du couplage de l'insert mobile (704) avec l'embase tibiale (502), le troisième élément de mise en prise (560) est également conçu pour venir en prise avec le premier bord (544) ou le deuxième bord (546) du premier élément de mise en prise (540) lors de la rotation de l'insert mobile (704) autour d'au moins le premier axe (542).

6. Système de la revendication 5, dans lequel la mise en prise du troisième élément de mise en prise (560) avec le premier bord (544) et le deuxième bord (546) du premier élément de mise en prise (540) limite la rotation de l'insert mobile (704).

7. Système de l'une quelconque des revendications 5 et 6, dans lequel le troisième élément de mise en prise (560) est formé dans une partie antérieure de l'insert mobile (704).

8. Système de l'une quelconque des revendications 4 à 7, dans lequel l'insert mobile (704) comprend un quatrième élément de mise en prise (570) conçu pour venir en prise avec le deuxième élément de mise en prise (550) de l'embase tibiale (502) lors de la rotation de l'insert mobile (704) autour d'un second axe par rapport à l'embase tibiale (502), et dans lequel la mise en prise du quatrième élément de mise en prise (570) avec le deuxième élément de mise en prise (550) limite la rotation de l'insert mobile (704) autour du second axe.

9. Système de la revendication 8, dans lequel le quatrième élément de mise en prise (570) est formé dans l'insert mobile (704) adjacent à un évidement conçu pour recevoir le montant de charnière (508).

10. Système de la revendication 9, dans lequel le quatrième élément de mise en prise (570) est postérieur à l'évidement conçu pour recevoir le montant de charnière (508).

11. Système de l'une quelconque des revendications 8 à 10, dans lequel la rotation de l'insert mobile (704) autour du second axe comprend l'adduction et l'abduction de l'ensemble prothèse.

12. Système de l'une quelconque des revendications 3 à 11, dans lequel l'insert fixe (904) est conçu pour venir en prise avec le premier élément de mise en prise (540) de l'embase tibiale (502) lors du couplage de l'insert fixe (904) avec l'embase tibiale (502).

13. Système de la revendication 12, dans lequel l'insert fixe (904) comprend un troisième élément de mise en prise (560) qui vient en contact avec le premier bord (544), le deuxième bord (546) et le troisième bord (548) du premier élément de mise en prise (540) pour empêcher la rotation de l'insert mobile (704) autour d'au moins le premier axe (542).

14. Système de l'une quelconque des revendications 12 et 13, dans lequel l'insert fixe (904) comprend un quatrième élément de mise en prise (570) formé dans une partie centrale de l'insert fixe (904), le quatrième élément de mise en prise (570) vient en contact avec le deuxième élément de mise en prise (550) de l'embase tibiale (502) lors du couplage de l'insert fixe (904) avec l'embase tibiale (502).

15. Système de la revendication 14, dans lequel le quatrième élément de mise en prise (570) comprend une paire de nervures qui viennent en prise avec le deuxième élément de mise en prise (550) sur des côtés opposés de celui-ci.
